# EUROPEAN PATENT SPECIFICATION

(11) **EP 1 722 811 B1**
(45) Date of publication and mention of the grant of the patent: **07.11.2007**
(21) Application number: 05720656.7
(22) Date of filing: 07.03.2005
(51) Int. Cl.: A61K 38/18

(54) **USE OF HEPATOCYTE GROWTH FACTOR FOR PROMOTING INDUCTION OF VASCULAR DIFFERENTIATION**
VERWENDUNG DES HEPATOZYTEN-WACHSTUMSFAKTORS ZUR FÖRDERUNG DER INDUKTION DER VASKULÄREN DIFFERENZIERUNG
UTILISATION DU FACTEUR DE CROISSANCE HEPATOCYTE POUR PROMOUVOIR L'INDUCTION D'UNE DIFFERENCIATION VASCULAIRE

(30) Priority: 09.03.2004 JP 2004066415
(43) Date of publication of application: 22.11.2006
(73) Proprietor: Tohoku Technoarch Co., Ltd., Sendai-shi, Miyagi 980-0845 (JP); Kringle Pharma Inc., Toyonaka-shi, Osaka 560-0082 (JP)
(72) Inventor: KUBO, Hiroshi, Sendai-shi, Miyagi 989-3201 (JP); ISHIZAWA, Kota c/o TOHOKU TECHNOARCH CO., LTD.,, Miyagi 980-0845 (JP); SASAKI, Hidetada, Sendai-shi, Miyagi 982-0841 (JP); NAKAMURA, Toshikazu, Kyoto-shi, Kyoto 606-8333 (JP)
(74) Representative: Kalhammer, Georg
(86) International application number: PCT/JP2005/004385
(87) International publication number: WO 2005/084700

(56) References cited:
- ISHIZAWA K ET AL: "Hepatocyte growth factor induces angiogenesis in injured lungs through mobilizing endothelial progenitor cells" BIOCHEMICAL AND BIOPHYSICAL RESEARCH COMMUNICATIONS, ACADEMIC PRESS INC. ORLANDO, FL, US, vol. 324, no. 1, 5 November 2004 (2004-11-05), pages 276-280, XP004605930 ISSN: 0006-291X
- GALIMI F ET AL: "HEPATOCYTE GROWTH FACTOR INDUCES PROLIFERATION AND DIFFERENTIATION OF MULTIPOTENT AND ERYTHROID HEMOPOIETIC PROGENITORS" JOURNAL OF CELL BIOLOGY, ROCKEFELLER UNIVERSITY PRESS, NEW YORK, US, US, vol. 127, no. 6, PART 1, 1 December 1994 (1994-12-01), pages 1743-1754, XP000564573 ISSN: 0021-9525
- BUSSOLINO F ET AL: "HEPATOCYTE GROWTH FACTOR IS A POTENT ANGIOGENIC FACTOR WHICH STIMULATES ENDOTHELIAL CELL MOTILITY AND GROWTH" JOURNAL OF CELL BIOLOGY, ROCKEFELLER UNIVERSITY PRESS, NEW YORK, US, US, vol. 119, no. 3, November 1992 (1992-11), pages 629-641, XP000615596 ISSN: 0021-9525
- TAKAI KENJI ET AL: "Hepatocyte growth factor is constitutively produced by human bone marrow stromal cells and indirectly promotes hematopoiesis" BLOOD, vol. 89, no. 5, 1997, pages 1560-1565, XP002327688 ISSN: 0006-4971
- XIN XIAOHUA ET AL: "Hepatocyte growth factor enhances vascular endothelial growth factor-induced angiogenesis in vitro and in vivo" AMERICAN JOURNAL OF PATHOLOGY, vol. 158, no. 3, March 2001 (2001-03), pages 1111-1120, XP002327689 ISSN: 0002-9440

## Description

### Technical Field

The present invention relates to the use of hepatocyte growth factor for the manufacture of a medicament for promoting induction of differentiation of bone marrow cells into endothelial progenitor cells or endothelial cells.

### Background Art

Hepatocyte growth factor (hereinafter referred to as HGF) was originally identified as a growth factor for mature hepatocytes, and its gene (cDNA) was cloned in 1989 (see non-patent literatures 1 and 2).

It has been revealed so far that HGF exerts various biological activities such as cell proliferation, promotion of cell migration, morphogenesis induction, cell death inhibition, and the like on various cells as well as hepatocytes (see non-patent literatures 3 to 6).

The biological activities of HGF are expressed via its receptor, i.e. c-Met tyrosine kinase. HGF has various biological activities and has functions of repairing and protecting various tissues from various injuries.

Angiogenesis promoting activity is one of the HGF functions of regeneration or protection of tissues. HGF not only stimulates proliferation and migration of vascular endothelial cells, but also shows in vivo potent angiogenesis-inducing activity (see non-patent literatures 7 to 10).

However, any one of the above-mentioned literatures does not describe that HGF promotes induction of differentiation of bone marrow cells into endothelial progenitor cells or endothelial cells.
[Patent literature 1] JP-A-89869/1996
[Patent literature 2] JP-A-172207/1994
[Non-patent literature 1] Biochemical and Biophysical Research Communications, 1984, vol. 122, p. 1450-1459
[Non-patent literature 2] Nature, 1989, vol. 342, p.440-443
[Non-patent literature 3] The Journal of Cell Biology, 1985, vol. 129, p.1177-1185
[Non-patent literature 4] The Journal of Biochemistry, 1986, vol. 119, p.591-600
[Non-patent literature 5] International Review of Cytology), 1999, vol.186, p.225-260
[Non-patent literature 6] Kidney International, 2001, vol. 59, p.2023-2038
[Non-patent literature 7] The Journal of Cell Biology, 1992, vol.119, p.629-641
[Non-patent literature 8] Proceedings of the National Academy of Sciences of the United States of America, 1993, vol. 90, p.1937-1941
[Non-patent literature 9] Circulation, 1998, vol. 97, p.381-390
[Non-patent literature 10] Hypertension, 1999, vol. 33, p.1379-1384

### Disclosure of Invention

Recently, concept of angiogenesis has been largely changed. That is, angiogenesis in adults has been considered to be resulted only from proliferation and migration of existing vascular endothelial cells, however, it is now suggested that there is a possibility that vascular regeneration of vasculogenesis type, which has been considered to occur only in the embryo stage, might take place as a result of angiogenesis that occurs when endothelial progenitor cells in blood flow are involved. Based on this assumption, the development of a therapeutic method for regeneration of blood vessels by application of endothelial progenitor cells or endothelial cells is expected today.

Since endothelial progenitor cells in adults are derived from bone marrow, an object of the present invention is to provide an agent for promoting induction of differentiation of bone marrow cells into endothelial progenitor cells or endothelial cells.

The present inventors have newly found that (1) expression of endothelial progenitor cells is increased in peripheral blood by HGF administration, (2) such increase is derived from bone marrow cells, and (3) HGF promotes induction of differentiation of bone marrow cells into endothelial progenitor cells or endothelial cells. On the basis of these findings, the present inventors have further studied and completed the present invention.

Namely, the present invention relates to the use of HGF for preparing a medicine for promoting induction of differentiation of bone marrow cells into endothelial progenitor cells or endothelial cells.

Furthermore, described herein is a method for culturing bone marrow cells together with an agent for induction of differentiation containing HGF, and transplanting the endothelial progenitor cells or endothelial cells differentiated from bone marrow cells into mammals, or a method for culturing bone marrow cells, transplanting the proliferated bone marrow cells into mammals and simultaneously administering an agent for promoting induction of differentiation containing HGF, and inducing differentiation of the transplanted bone marrow cells into endothelial progenitor cells or endothelial cells.

Still furthermore, described herein is a gene medicine for promoting induction of differentiation of bone marrow cells into endothelial progenitor cells or endothelial cells, comprising introduction of HGF gene as well as administration of HGF.

The agent for promoting induction of vascular differentiation according to the present invention accelerates induction of differentiation of bone marrow cells into endothelial progenitor cells or endothelial cells. Endothelial progenitor cells are differentiated into endothelial cells, and the endothelial cells exhibit effects resulting in vasculogenesis type angiogenesis or vascular regeneration. Since the agent of the present invention promotes induction of differentiation of bone marrow cells into endothelial progenitor cells or endothelial cells, it is useful for treatment of diseases which require angiogenesis or vascular regeneration.

Further, by culturing bone marrow cells in the presence of an agent for promoting induction of differentiation according to the present invention, endothelial progenitor cells or endothelial cells differentiated from the bone marrow cells can be produced. The endothelial progenitor cells or endothelial cells obtained as such can be utilized as a transplantation cells for angiogenesis or vascular regeneration in the field of regenerative medicine.

### Brief Explanation of the Drawing

Fig. 1 shows expression distribution of FlK-1 antibodies in bone marrow-derived cell fraction of peripheral blood of recipient mice with injured pulmonary capillaries.
Fig. 2 shows histological findings of lung of recipient mice with injured pulmonary capillaries.
Fig. 3 shows immunofluorescence staining of pulmonary capillaries in recipient mice with injured pulmonary capillaries.

### Best Mode for Carrying Out the Invention

HGF used in the present invention is a known substance. HGF prepared by various processes can be used in the present invention as long as it is purified enough to be used as a medical agent. Regarding production processes of HGF, for example, HGF can be obtained by cultivating primary culture cells or cells of an established cell line which produce HGF, isolating HGF from culture supernatant or the like and purifying the isolated HGF. Alternatively, recombinant HGF can also be obtained according to a genetic engineering technique by integrating a gene encoding HGF into an appropriate vector, inserting the vector into proper host cells for transformation thereof and collecting the target recombinant HGF from the culture supernatant of the transformed cells (see, for example, JP-A-111382/1993; Biochem. Biophys. Res. Commun., 1989, vol. 163, p.967).

The above-mentioned host cells are not particularly limited, and various kinds of host cells conventionally used in the genetic engineering techniques, such as Escherichia coli, yeast, animal cells with the exception of human embryonic stem cells or the like may be used. The obtained HGF, so long as it has substantially the same action as natural HGF, may include substitution, deletion, addition and/or insertion of one or more (e.g., several) of amino acids in the amino acid sequence thereof. Similarly, HGF may include substitution, deletion and/or addition of sugar chain (s). Here "the deletion, substitution, addition or insertion of one or more of amino acids" in the amino acid sequence means that the amino acids in a number that can naturally occur (one to several amino acids), may be deleted, substituted, added and/or inserted into the amino acid sequence by known technical methods such as genetic engineering techniques, site-specific mutation induction and the like.

Also, "HGF including substitution, deletion and/or addition of sugar chain(s)" means, for example, (1) glycosylation-deficient HGF which is obtained by treating a natural glycosylated HGF with an enzyme to remove sugar chain (s), (2) HGF of which amino acid sequence is mutated at a glycosylation site to inhibit glycosylation, or (3) HGF of which amino acid sequence is mutated in such a manner that glycosylation occurs at a site different from the natural glycosylation site.

Further, HGF also includes a protein having at least 60 % or more homology, preferably 80 % or more homology, more preferably 90 % or more homology, still more preferably 95 % or more homology to the amino acid sequence of HGF and also having an activity to induce differentiation of bone marrow cells into endothelial progenitor cells or endothelial cells. The above-mentioned "homology" between amino acid sequences generally means the level of homology between the amino acid residues constituting the amino acid sequences when the primary structures of the proteins are compared.

HGF used in the present invention may have a carboxylate (-COO⁻), amide (-CONH₂) or ester (-COOR) as well as a carboxyl group (-COOH) at its C terminal so long as it has substantially the same actions as natural HGF. Here, as R in the ester, optionally substituted lower alkyl groups (e.g., methyl, ethyl, propyl, cyclopentyl, benzyl, phenethyl, etc.), aryl groups (e.g., phenyl, α-naphthyl, etc.), pivaloyloxymethyl groups which are generally used as an ester for oral administration and the like may be mentioned. In addition, HGF usable in the present invention may include HGF in which an amino group of a methionine residue at the N-terminal is protected by a protective group (e.g., an acyl group such as formyl, acetyl, etc.), HGF in which a glutamyl group produced by cutting an N-terminal side in vivo has changed to a pyroglutamic acid and the like.

In the present invention, "endothelial progenitor cells" mean cells that differentiate into endothelial cells, mainly into vascular endothelial cells.

Since the agent for promoting induction of differentiation according to the present invention accelerate s induction of differentiation of bone marrow cells into endothelial progenitor cells or endothelial cells which participate in vasculogenesis type angiogenesis or vascular regeneration, it can be applied to diseases which require angiogenesis or vascular regeneration in mammals (e.g. cow, horse, pig, sheep, dog, cat, etc.) including human beings. Such diseases include, for example, lower limb arteriosclerosis obliterans, myocardial infarction, ischemic enteritis, ischemic colitis, cerebral infarction, and the like. Since angiogenesis or vascular regeneration is one of physiological phenomena in vasculogenesis which are observed in the healing stage of burns or wounds, the agent for inducing vascular differentiation according to the present invention is also useful in the treatment of burns and wounds.

Further, since endothelial progenitor cells differentiate into endothelial cells, they are involved in the repair of injured vascular endothelium. Thus, the agent according to the present invention for inducing vascular differentiation can be used for the repair and regeneration of vascular endothelium in diseases which are associated with vascular endothelial injury, such as disseminated intravascular coagulation; platelet thrombus formation in small vessels; thrombosis; arteriosclerosis; diabetic arteriosclerosis; vascular endothelial injury due to drugs such as anti-tumor agents (e.g. doxorubin, etc.); vascular endothelial injury due to smoking toxicity; autoimmune diseases such as rheumatic arthritis, polymyositis and polyarteritis; vascular endothelial injury caused by complications due to malignant diseases such as lymphoma, leukemia, stomach cancer, etc.; vascular endothelial injury caused by bacterial or viral infections or by vaccination.

The agent for promoting induction of vascular differentiation according to the present invention may take various dosage forms such as liquid preparations, solid preparations, capsules and the like, however, it is generally formulated with HGF alone or with a combination of HGF and a conventional carrier into injections, inhalations, suppositories or orally administrable preparations. The above-mentioned injections may be aqueous injections or oily injections.

In the case of aqueous injections, they can be prepared in such a manner that HGF is dissolved in, for example, a solution prepared by appropriately adding a pharmaceutically acceptable carrier such as isotonic agents (e.g. sodium chloride, potassium chloride, glycerin, mannitol, sorbitol, boric acid, borax, glucose, propylene glycol, etc.), buffers (e.g. phosphoric acid buffer, acetic acid buffer, boric acid buffer, carbonic acid buffer, citric acid buffer, Tris-buffer, glutamic acid buffer, ε-aminocarproic acid buffer, etc.), preservatives (e.g. methyl p-oxybenzoate, ethyl p-oxybenzoate, propyl p-oxybenzoate, butyl p-oxybenzoate, chlorobutanol, benzyl alcohol, benzalkonium chloride, sodium dehydroacetate, sodium edetate, boric acid, borax, etc.), thickners (e.g. hydroxyethylcellulose, hydroxypropylcellulose, polyvinyl alcohol, polyethylene glycol, etc.), stabilizers (e.g. sodium hydrogensulfite, sodium thiosulfate, sodium edetate, sodium citrate, ascorbic acid, dibutyl hydroxy toluene, etc,.), pH control agents (e.g. hydrochloric acid, sodium hydroxide, phosphoric acid, acetic acid, etc.) and the like to an aqueous solvent (e.g. injectable water, purified water, etc.), and then the solution is filtered through a filter, etc. , sterilized and filled in a sterile container according to a known method. Further, appropriate solubilizers such as alcohols (e.g. ethanol, etc.), polyalcohol (e.g. propylene glycol, polyethylene glycol, etc.) or non-ionic surfactants (e.g. polysorbate 80, polyoxyethylene hydrogenated castor oil 50, etc.) may be used.

In the case of oily preparations, sesame oil, soy bean oil and the like are used as an oily solvent, and benzyl benzoate, benzyl alcohol and the like may be used as a solubilizer. The obtained injectable solution is usually filled in an ampoule or a vial. The HGF content in the injection preparation is usually adjusted to about 0.0002 to 0.2 w/v %, preferably about 0.001 to 0.1 w/v %. It is preferable that liquid preparations such as injections are preserved after removing water by freeze-preservation or freeze-drying. The freeze-dried preparation is redissolved in injectable distilled water before use.

In the case of orally administrable preparations, the dosage forms include, for example, tablets, granules, fine granules, powders, capsules, liquids, emulsions, suspensions, syrups and the like. These preparations may be prepared by the conventional method. In the case of granules or tablets, they can be produced by using pharmaceutically acceptable additives such as excipients (e.g. lactose, white sugar, glucose, starch, crystalline cellulose, etc.), lubricants (e.g. magnesium stearate, talc, stearic acid, calcium stearate, etc.), disintegrators (e.g. starch, carmellose sodium, calcium carbonate, etc.), binders (e.g. starch paste, hydroxypropyl cellulose solution, carmellose solution, gum arabic solution, gelatin solution, sodium alginate solution, etc.), and the like. Also, granules or tablets may be coated with an appropriate coating agent (e.g. gelatin, white sugar, gum arabic, carnauba wax, etc.), enteric coating agents (e.g. cellulose acetate phthalate, methacrylic acid copolymer, hydroxypropyl cellulose phthalate, carboxymethyethyl cellulose, etc.), and the like.

In the case of capsules, known excipients such as magnesium stearate, calcium stearate, talc and light anhydrous silicic acid for enhancing flowability and lubricability; crystalline cellulose or lactose for increasing flowability under pressure; and the above-mentioned disintegrators may be appropriately selected. HGF is homogeneously blended or granulated with the above-mentioned excipient, or the granules may be coated with a suitable coating agent and then filled in a capsule, or the granules may be encapsulated with a capsule base (e.g. gelatin) having increased plasticity which is endowed with addition of glycerin, sorbitol, etc. If required, coloring agents or preservatives (e.g. sulfur dioxide, methyl p-oxybenzoate, ethyl p-oxybenzoate, propyl p-oxybenzoate, butyl p-oxybenzoate, etc.) may be added to the capsule preparation. The capsule preparation may take a form of enteric coated capsule, gastric acid-resistant capsule, release-controlled capsule or the like in addition to conventional capsule preparations.

In the case of enteric coated capsule preparations, HGF coated with an enteric coating agent or HGF to which the above-mentioned suitable excipients are added is filled in a conventional capsule. Alternatively, HGF alone or HGF to which the above-mentioned suitable excipients are added may be encapsulated in an enteric coated capsule or in a capsule formed from a base material comprising an enteric polymer.

In the case of syrups, stabilizers (e.g. sodium edetate, etc.), suspending agents (e.g. gum arabic, carmellose, etc.), corrigents (e.g. simple syrup, glucose, etc.), perfumes, and the like can be appropriately selected and used.

Further, suppositories can be prepared by the conventional formulation method using a conventional suppository base (e.g. cacao butter, lauric oil, glycerogelatin, macrogol, Witepsol, etc.) and the like.

Furthermore, inhalation preparations may be produced by the conventional formulation method. In the formulation of inhalations, any additives may be used so long as they are commonly utilized for inhalation preparations. For example, in addition to propellants, the above-mentioned excipients, binders, lubricants, preservatives, stabilizers, isotonic agents, pH control agents and corrigents (e.g. citric acid, menthol, ammonium glycyrrhizate, glycine, perfume, etc.) are used. As the propellants, liquefied gas propellants, compressed gas and the like are used. The liquefied gas propellants include, for example, fluorinated hydrocarbons such as alternative chlorofluorocarbons (e.g. HCFC-22, HCFC-123, HCFC-134a, HCFC-142, etc.), liquefied petroleum ether, dimethyl ether, and the like. The compressed gas includes, for example, soluble gas (e.g. carbon dioxide gas, nitrous oxide gas, etc.) and inert gas (e.g. nitrogen gas, etc.).

Moreover, HGF used in the present invention together with a biodegradable polymer may be formulated into a delayed release preparation. In the delayed release preparations of HGF, such effects as maintenance of blood level, reduction of administration frequency, alleviation of adverse effects, etc. may be expected. Such delayed release preparations can be prepared, for example, according to the conventional method as described in Drug Delivery System, chapter 3, 1986 (published by CMC, Japan). The biodegradable polymer used in the present invention may be appropriately selected from known biodegradable polymers, and include, for example, polysaccharides (e.g. starch, dextran, chitosan, etc.), proteins (e.g. collagen, gelatin, etc.), polyamino acids (e.g. polyglutamic acid, polylysine, polyleucine, polyalanine, polymethionine, etc.), polyesters (e.g. polylactic acid, polyglycolic acid, lactic acid/glycolic acid polymer or copolymer, polycaprolactone, poly-β-hydroxybutyric acid, polymalic acid, poly-acid anhydride, fumaric acid-polyethylene glycol-vinylpyrrolidone copolymer, etc.), poly(ortho esters), polyalkyl-cyanoacrylic acids (e.g. polymethyl-α-cyanoacrylic acid, etc.), polycarbonates (e.g. polyethylene carbonate, polypropylene carbonate, etc.), among which polyesters are preferable, and polylactic acid, lactic acid/glycolic acid polymers or copolymers are more preferable. When a polylactic acid/glycolic acid polymer or copolymer is used, its component ratio (mol %) of lactic acid/glycolic acid is about 100/0 to 50/50 in case of the delayed release time of two weeks to three months, preferably two weeks to one month, though such ratio depends on the delayed release time. The average molecular weight of such polylactic acid/glycolic acid polymer or copolymer is generally 5,000 to 20,000. The polylactic acid/glycolic acid polymer or copolymer can be prepared according to the known method, for example, the method as described in JP-A-28521/1986. Although there is no limitation with respect to mixing ratio of HGF with biodegradable polymer, HGF is used generally in an amount about 0.01 w/w % to 30 w/w % relative to the biodegradable polymer.

HGF content in the above-mentioned preparations may be appropriately adjusted depending on dosage form, indications, degree of diseases, age, etc.

The agent for promoting induction of vascular differentiation of the present invention may contain appropriately other medicinal active ingredients, so long as they are not contrary to the purpose of the present invention. Examples of such active ingredients include, for example, coronary vasodilators (e.g. amyl nitrite, isosorbid nitrate, nitroglycerin, trapidil, etc.), β-blockers (e.g. oxprenolol, carteolol, bucumolol, bufetolol, propranolol, pindolol, etc.), calcium antagonists (e.g. diltiazem, verapamil, nifedipine, nicardipine, etc.), agents for peripheral circulation disorders (e.g. alprostadil alfadex, kallidinogenase, tocopherol, nicomol, etc.), antiarrhythmic agents (e.g. adimarin, procaineamide, lidocaine, etc.), hypo tensive agents (e.g. furosemide, trichloromethiazide, hydralazine, sympatholytic agents, calcium antagonists, etc.), antilipemic agents (e.g. clofibrate, pravastatin, simvastatin, lovastatin, nicomol, etc.), anticoagulants (e.g. heparin, warfarin, dicumarol, aspirin, etc.), thrombolytic agents (e.g. urokinase, etc.), antidiabetic agents (e.g. tolbutamide, chilorpropamide, acetahexamide, glibenclamide, metformin, acarbose, etc.), antiinflammatory agents (e.g. diclofenac sodium, ibuprofen, indomethacin, etc.), antibiotics (e.g. cefixime, cefdinir , ofloxacin, tosfloxacin, etc.), antimycotics (e.g. fluconazole, itraconazole, etc.), and the like.

Also, a preparation containing those active ingredients may be used in combination with a preparation of the present invention. There is no limitation on those medicinal active ingredients so long as the purpose of the present invention can be attained, and it is possible to use such active ingredients appropriately in a suitable mixing ratio or combination ratio.

The agents for promoting induction of vascular differentiation of the present invention may be administered via a suitable administration route depending on their dosage form. For example, injections may be administered via intravenous, intraarterial, subcutaneous or intramuscular route and the like. The injection dose is usually 0.001 mg to 1000 mg on the basis of HGF, preferably 0.01 mg to 100 mg, which is appropriately administered once a day or several times a day in a divided manner, although it may be appropriately adjusted depending on the condition, age, body weight of patients, etc.

The agents for promoting induction of vascular differentiation of the present invention can be used in vitro for promoting induction of differentiation of bone marrow cells into endothelial progenitor cells or endothelial cells. As the bone marrow cells, any bone marrow cells of mammals including human beings can be used, while it is preferable to use floating bone marrow cells. For example, human bone marrow cells are harvested by the known method, suspended in a cell culture liquid, seeded in a plastic petri dish, and cultured to collect only floating cells therefrom. Subsequently, the floating bone marrow cells are cultured together with an agent for promoting induction of vascular differentiation of the present invention. As the cell culture liquid, a conventional culture liquid such as DMEM, MEM, RPMI1640, IMDM, etc., can be used. Although additives which are commonly used in cell culture, such as fetal bovine serum, may be added to the above-mentioned cell culture liquid, it is preferable to use a serum-free cell culture liquid in view of transplantation immunology. HGF concentration in the agent for promoting induction of vascular differentiation is about 1 ng/mL to about 100 ng/mL. Culture conditions are those employed in the usual cell culture, for example, at about 35°C ± 2°C in the presence of 5 % carbon dioxide and the like. The bone marrow cells cultured as such are cultured for one to five weeks, resulting in differentiation into endothelial progenitor cells or endothelial cells. The differentiation-induced endothelial progenitor cells or endothelial cells derived from the bone marrow cells obtained as above are available as a cell for organ transplantation.

To be more specific, endothelial progenitor cells or endothelial cells differentiated and proliferated from bone marrow cells of a patient with lower limb arteriosclerotic obliterans can be, for example, intramuscularly injected for transplantation to necrotizing ulcerative part of the patient's body caused by lower limb arteriosclerotic obliterans. According to this procedure, it is possible to obtain a large amount of endothelial progenitor cells or endothelial cells needed for transplantation in patients with lower limb arteriosclerotic obliterans, from the bone marrow cells of said patients themselves.

Further, the transplantation may include another embodiment which comprises culturing bone marrow cells, utilizing the proliferated, undifferentiated bone marrow cells as a cell for transplantation, and administering an agent for promoting induction of vascular differentiation of the present invention to a recipient. According to the present procedure, since a small amount of bone marrow cells harvested from patients with lower limb arteriosclerotic obliterans is cultured and proliferated, and a large amount of the resultant bone marrow cells is returned to the patient at the same time when an agent for promoting vascular differentiation according to the present invention is administered, induction of differentiation on transplanted bone marrow cells into endothelial progenitor cells or endothelial cells occurs effectively in the body.

With respect to the bone marrow cells to be used for the above-mentioned transplantation, they are preferably those collected from the same recipients to be transplanted, in view of transplantation immunology.

In recent years, gene therapies using HGF gene have been reported (see Circulation, 1997, vol. 96, No. 3459; Nature Medicine, 1999, vol. 5, p. 226-230; Circulation, 1999, vol. 100, No. 1672; Gene Therapy, 2000, vol. 7, p. 417-427), and such gene therapies have been technologically established. The present invention includes a gene therapy agent comprising introduction of HGF gene for induction of differentiation of bone marrow cells into endothelial progenitor cells or endothelial cells, as well as administration of HGF as mentioned above. Hereinafter, HGF gene therapy will be described in detail.

As used herein, "HGF gene" means a gene capable of expressing HGF. To be more specific, there is exemplified a gene wherein cDNA of HGF is integrated in a suitable expression vector (nonvirus vector, virus vector) as described in non-patent literature 2; Japanese patent No. 2,777,678; Biochem. Biophys. Res. Commun., 1989, vol. 163, p.967; or Biochem. Biophys. Res. Commun., 1990, vol. 172, p. 321. Here, the base sequence of cDNA encoding HGF is described in the above literatures, and also registered in databases such as Genebank. Based on the above sequence data, it is therefore possible to carry out, for example, RT-PCR to mRNA from liver, etc., by use of a proper DNA segment as a PCR primer, whereby cDNA cloning of HGF is performed. Such cloning could be easily done by those skilled in the art according to fundamental textbooks such as Molecular Cloning 2nd Edit., published by Cold Spring Harbor Laboratory Press (1989).

Further, the HGF gene used in the present invention is not limited to the above-mentioned genes, and any HGF gene can be used as the HGF gene of the present invention so long as it expresses a protein having the substantially the same activity with that of HGF. That is, among DNAs which can hybridize with the above-mentioned cDNA under stringent conditions and DNAs encoding a protein wherein one to plural amino acids (preferably several amino acids) in the amino acid sequence encoded by the above-mentioned cDNA are substituted, deleted, added and/or inserted, any DNA encoding a protein having an activity of HGF is included in the scope of the HGF gene used in the present invention. Such DNAs can be easily obtained by common hybridization method, PCR method and the like, specifically by reference to fundamental textbooks such as Molecular Cloning mentioned above.

HGF genes used in the present invention can be applied to induction of differentiation of bone marrow cells into alveolar cells, in the same manner as in the above-mentioned HGF protein.

When a gene therapy agent comprising HGF gene as an active ingredient is administered to a patient, such administration can be conducted in the conventional manner described in, for example, Basic Technology of Gene Therapy, Separate Volume of Experimental Medicine published by Yodosha, Japan, 1996; Gene Introduction and Expression Analysis Method, Separate Volume of Experimental Medicine, published by Yodosha, Japan, 1997; Gene Therapy Development Research Handbook, edited by The Japan Society of Gene Therapy, published by NTS, Japan, 1999.

The dosage form may include a variety of known forms suitable for each administration form mentioned above. DNA content in the preparation can be appropriately controlled, depending on the target disease, age and body weight of patients, etc., and usually the DNA content in accordance with the pres ent invention is 0.0001 to 100 mg, preferably 0.001 to 10 mg.

Further, HGF gene and HGF may be used each independently or in combination.

The present invention will be illustrated by the following Examples, however it is not limited thereto. The percentage (%) means % by mass unless otherwise indicated.

### Example 1

### Recipient Mice

Mice transgenic for enhanced green fluorescent protein (GFP) on a C57BL/6 strain background were established at Osaka University, Japan. Liver cells of fetal livers harvested from day 13.5 GFP embryos were transplanted to C57BL/6 male mice which were irradiated using doses of 12 Gy before the transplantation, according to the method by Morishita, et. al as described in Hypertension, 1999, vol. 33, p.1379-1384. Those mice in which over 95 % of the circulating white blood cells (peripheral leukocytes) were completely replaced with bone marrow cells of GFP mouse origin three weeks after the transplantation were served as recipient mice in the experiment.

### Induction of pulmonary capillary injury and treatment

Porcine pancreas elastase (200 units/kg, Sigma, St Louis, MO) was instilled intranasally into recipient mice. Three weeks after the elastase instillation, the recipient mice exhibited emphysematous changes in the lungs, and injured pulmonary capillaries were observed. At this point, the recipient mice with induced pulmonary emphysema were randomly divided into two groups, i.e. vehicle administration group and HGF administration group, which were injected intraperitoneally with a balanced diet and with HGF respectively, at a dose of 1 mg/kg once daily for 12 days.

### Histological anatomy

Lungs of the recipient mice were fixed with 4% paraformaldehyde-PBS (phosphate buffered saline) at a pressure of 20 cm H₂O, and paraffin-embedded sections were prepared according to the conventional method, and stained with hematoxylin-eosin (HE). GFP was detected using anti-GFP antibody (Abcam, Cambridge, UK) and visualized with 3,3'-diaminobenzidine.

For the purpose of identifying the phenotype of GFP-positive cells, immunofluorescence staining of the frozen sections was performed using anti-CD34 and anti-CD45 antibodies (Pharmingen, San Diego, CA).

Sca-1⁺ peripheral blood cells (surface antigen derived from bone marrow cells) collected from each mouse were isolated using the Magnetic Cell Sorting System with anti-Sca-1 coated microbeads (Miltenvi Biotec, Bergisch Gladbach, Germany). Cell distribution of endothelial progenitor cells or endothelial cells in the isolated Sca-1⁺ peripheral blood was measured by flow cytometry using a FACS Calibur (automated cell analysis and sorting apparatus, BD, San Jose, CA), wherein expression of Sca-1 and Flk-1 which is one of receptors for vascular endothelial growth factor (VEGF) was used as an index. Sca-1 was detected using fluoresein isothiocyanate-labeled anti-Sca-1 antibody, and Flk-1 was detected using phycoerythrin (fluorescent red pigment)-labeled anti-Flk-1 antibody (Pharmingen).

### Results

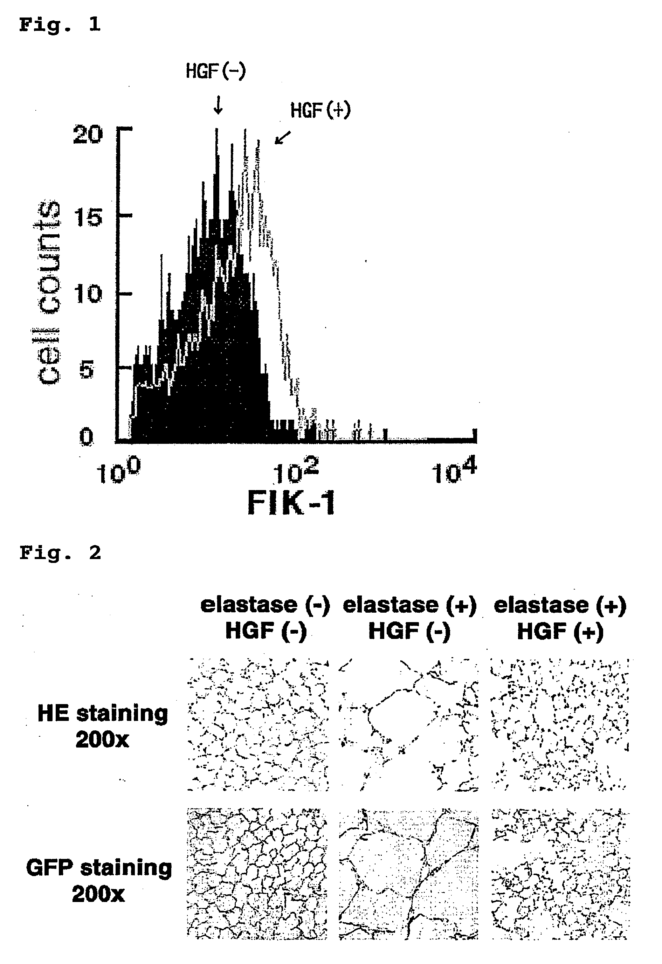

Distribution of Flk-1 positive cells in the recipient mice whose pulmonary capillaries are injured is shown in Fig. 1. The whole cell distribution in HGF administration group [HGF(+); gray line] was shifted to the right compared to that of control group [HGF(-)]. This result indicates that Flk-1 positive cells, i.e. endothelial progenitor cells or endothelial cells are increased in the HGF administration group.

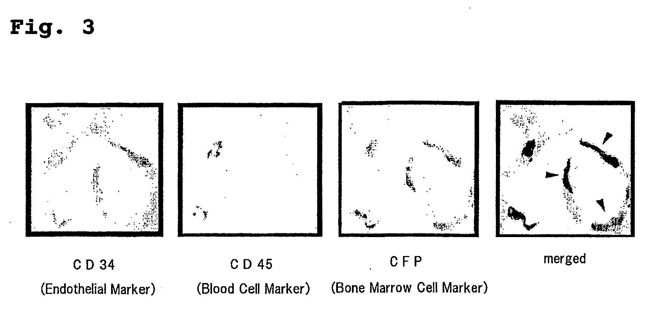

In the observations of lung tissue sections of the recipient mice, pulmonary capillaries in the recipient mice which had received elastase were destroyed, while in the HGF administration group, pulmonary capillaries were observed to almost the same extent with lung of the recipient mice in the control group receiving no elastase (see Fig. 2). The results of histological observation of the recipient mice by immunofluorescence staining showed that pulmonary capillary endothelial cells in the HGF administration group were differentiated from GFP mice-derived bone marrow cells (see Fig. 3).

This result indicated that endothelial progenitor cells or endothelial cells wherein differentiation from bone marrow cells was induced were further differentiated to newly regenerate pulmonary capillaries.

### Example 2

### Methods

A total of 0.5-1.0 ml of peripheral blood was obtained from each mouse. Peripheral blood mononuclear cells (PBMCs) were separated by a Ficoll-Hypaque density gradient (Lymphosepal; IBL, Gunma, Japan). Red blood cells were depleted by RBC lysis buffer (0.15M NH₄Cl, 0.01M KHCO₃, 0.1mM EDTA-2Na, pH 7.2). Sca-1⁺/Flk-1⁺/ c-kit⁺ cells, consistent with peripheral EPCs, were staind with FITC-labeled anti-murine Sca-1 antibody (BD Pharmingen, San Diego, CA), PE-labeled anti-murine fetal liver kinase 1/vascular endothelial growth factor receptor-2 (Flk-1/VEGF-R2) (BD Pharmingen), and APC-labeled anti-c-kit antibody (BD Pharmingen) and analyzed by a FACSCalibur (BD, San Jose, CA).

### Results

The number of PBMCs, which contain an EPC population, was increased by HGF treatment compared with saline-treated mice. The number of circulating Sca-1⁺/Flk-1⁺/c-kit⁺ cells increased in HGF-trated mice (Table 1). These results suggested that HGF induced an increase in the number of the peripheral Sca- 1⁺/Flk- 1⁺/c-kit⁺ cells, consistent with EPC phenotype, from bone marrow into the circulation, and that lung parenchyma could therefore receive more progenitor cells from the bone marrow.

**Table 1. Number of PBMCs and Sca-1⁺/Flk-1⁺/c-kit⁺ cells in saline- and HGF-treated mice.**

| treatment | PBMCs | Sca-1⁺/Flk-1⁺/c-kit⁺ cells |
|---|---|---|
| saline | 21.7 ± 1.7 | 1.1 ± 0.1 |
| HGF | 63.3 ± 8.8* | 7.6 ± 1.1* |

| | | |
|---|---|---|
| Values represent mean ± SEM x 10⁵ cells/mL. *Significantly different from saline-treated mice (P < 0.05). | | |

### Industrial applicability

The agent for promoting induction of vascular differentiation of the present invention is useful as a medicinal agent for promoting induction of differentiation of bone marrow cells into endothelial progenitor cells or endothelial cells. Since angiogenesis or vascular regeneration takes place using such endothelial progenitor cells or endothelial cells, the agent for promoting induction of vascular differentiation according to the present invention can be utilized in the field of regenerative medicine.

## Claims

1. Use of hepatocyte growth factor for the preparation of a medicament for promoting induction of differentiation of bone marrow cells into endothelial progenitor cells or endothelial cells.

## Patentansprüche

1. Verwendung eines Hepatozytenwachstumsfaktors für die Herstellung eines Medikaments zur Förderung der Induktion der Differenzierung von Knochenmarkzellen zu Endothelvorläuferzellen oder Endothelzellen.

## Revendications

1. Utilisation du facteur de croissance hépatocyte pour la préparation d'un médicament pour promouvoir l'induction d'une différenciation des cellules de la moelle osseuse en des cellules progénitrices endothéliales ou des cellules endothéliales.
